# NEUE EUROPÄISCHE PATENTSCHRIFT

(11) **EP 0 544 237 B2**
(45) Veröffentlichungstag und Bekanntmachung des Hinweises auf die Entscheidung über den Einspruch: **23.04.2003**
(45) Hinweis auf die Patenterteilung: 01.04.1998
(21) Anmeldenummer: 92120003.6
(22) Anmeldetag: 25.11.1992
(51) Int. Cl.: G01N 33/487

(54) **Vorrichtung zum Messen der Ionenkonzentration in fliessfähigen Medien**
Apparatus for measuring the ion concentration in flowable media
Appareil pour la mesure de la concentration des ions dans des milieux liquides

(30) Priorität: 28.11.1991 DE 4139121
(43) Veröffentlichungstag der Anmeldung: 02.06.1993
(73) Patentinhaber: Petermann, Heike, 91054 Erlangen (DE); Fenzlein, Paul-Gerhard, 90431 Nürnberg (DE)
(72) Erfinder: Fenzlein, Paul-Gerhard, W-8500 Nürnberg 80 (DE)
(74) Vertreter: Meissner, Bolte & Partner

(56) Entgegenhaltungen:
- EP-A- 0 079 086
- EP-A- 0 306 158
- WO-A-85/02257
- WO-A-87/06342
- WO-A-89/04481
- US-A- 4 786 394
- US-A- 5 004 538

## Beschreibung

Die Erfindung betrifft eine Vorrichtung zum Messen der lonenkonzentration in fließfähigen Medien und insbesondere in Körperflüssigkeiten nach dem Oberbegriff des Patentanspruches 1.

Bekannte Vorrichtungen zum Messen weisen als stationäre Analysegeräte eine Durchflußmeßzelle mit mehreren, auf die jeweils zu messende lonenart abgestimmten Meßelektroden, sowie eine Bezugselektrode und eine Meßwerterfassungs- und Auswerteeinrichtung auf, die mit den Meßelektroden sowie der Bezugselektrode elektrisch verbunden ist. Eine Anzeigeeinheit dient zur Anzeige der von der Meßwerterfassungs- und Auswerteeinheit ermittelten Konzentrationswerte der einzelnen lonenarten. Eine Steuereinheit etwa in Form eines üblichen Mikroprozessors mit Arbeits-, Daten-, Programmspeicher und einer zentralen Prozeßrecheneinheit dient zur Steuerung der vorrichtungsinternen Betriebsabläufe.

Nachteilig bei diesen bekannten Analysegeräten ist, daß jeweils nur die lonenarten analysierbar sind, auf die die Meßelektroden in der Durchtlußmeßzelle abgestimmt sind. Das Gerät ist also nicht oder nur durch komplizierte Umrüstungsarbeiten an variierende Analyseaufgaben anpaßbar.

Weiterhin ist aus der DE 37 25 597 C2 ein Meßsystem zum Messen der Konzentrationen unterschiedlicher lonenarten bekannt, bei dem ein Sensor mit mehreren, auf die jeweils zu messende lonenart abgestimmten Meßelektroden und ggfls. einer Bezugselektrode an einem als Handgriff ausgebildeten Träger auswechselbar angeordnet ist. Die Elektroden stehen über eine flexible Kabelverbindung mit einer Rechnerelektronik in Verbindung, in der die Meßwerterfassung und -auswertung sowie die Anzeige der ermittelten lonenkonzentrationen stattfinden. Durch die Auswechselbarkeit der Sensoren kann die in dieser Druckschrift gezeigte Vorrichtung an unterschiedliche Meßaufgaben angepaßt werden. Die Messung selbst erfolgt jedoch durch direkten in-vivo-Kontakt des Sensors mit einem dünnen Flüssigkeitsfilm, der menschliche und tierische Organe und Gewebe überzieht bzw. durch Eintauchen des Sensors in eine zu analysierende Flüssigkeit. Eine im Analysegerät sitzende Durchflußmeßzelle, in die eine Probe des zu analysierenden fließfähigen Mediums eingebracht wird, ist nicht vorgesehen.

Aus der EP-A-0 306 158 ist eine Wegwerfkartusche zur Verwendung in einer klinischen Meßvorrichtung bekannt, die mit einem Basisgerät verbindbar ist. Dabei werden über elektrische Kontakte elektrische Signale dem Basisgerät zugeführt, die durch Detektoren in der Kartusche nach Benutzung durch Kalibrierflüssigkeiten und/oder zu untersuchende klinische Flüssigkeiten erzeugt werden. Die Kartusche ist nur auf spezielle Meßgrößen ausgerichtet und zum einmaligen. Gebrauch bestimmt.

Der Erfindung liegt die Aufgabe zugrunde, eine Vorrichtung der gattungsgemäßen Art derart weiterzubilden, daß sie schnell und ohne großen Aufwand an unterschiedlichste Meßaufgaben anpaßbar ist. Eine Vorrichtung gemäß Oberbegriff des Patentanspruchs 1 ist bekannt (US 5 004 583 A).

Diese Aufgabe wird durch die im Kennzeichnungsteil des Patentanspruches 1 angegebenen Merkmale gelöst. Durch die Integration der Durchflußmeßzelle in einen für mehrere Probenflügkeiten verwendbaren Meßeinsatz, der in ein die restlichen Komponenten der Vorrichtung umfassendes Basisgerät auswechselbar einsetzbar ist, können Meßeinsätze mit unterschiedlichen Durchflußmeßzellen bereitgestellt werden, die jeweils auf unterschiedliche lonenarten abgestimmte Meßelektroden aufweisen. Für eine bestimmte Analyseaufgabe braucht dann jeweils nur der entsprechend richtige Meßeinsatz in das Basisgerät eingesetzt werden, womit die Analysevorrichtung sofort betriebsbereit ist. Damit ist die erfindungsgemäße Vorrichtung besonders vorteilhaft mehrfach verwendbar und in solchen Fällen einzusetzen, wo es auf eine besonders schnelle Anpassung an unterschiedlichste Aufgaben ankommt. Als Beispiel wird die Unfall-Notversorgung und insbesondere der Einsatz in einem Notarzt- oder Rettungswagen genannt.

Die in Anspruch 1 angegebenen Kodierung-Maßnahmen ermöglichen es, daß die erfindungsgemäße Vorrichtung automatisch jeden Meßeinsatz identifizieren kann. Dies bedeutet, daß das Basisgerät über eine Kodierung des Meßeinsatzes beispielsweise erkennt, auf weiche lonenarten die in dem speziellen Meßeinsatz befindlichen Meßelektroden abgestimmt sind. Damit kann das Basisgerät selbstätig die über den Meßeinsatz gemessenen Potentialwerte durch entsprechende in der zentralen Steuereinheit gespeicherte Umrechnungsfaktoren auf die jeweiligen lonenkonzentrationen umrechnen und zur Anzeige bringen. Hierdurch wird die Gefahr einer Fehlmessung auch bei schnellem Durchführen einer Messung im Notfall wesentlich reduziert.

Zur Kodierung ist ein in den Meßeinsatz integrierter PROM-Speicher verwendet, auf dessen gespeicherte Identifikationsinformation über eine nach dem Einsetzen des Meßeinsatzes in das Basisgerät hergestellte elektrische Geräteverbindung Zugriff genommen werden kann.

Es ist darauf hinzuweisen, daß die Kodierung nicht nur eine Identifikation der jeweiligen Meßelektroden ermöglicht. Darüberhinaus ist jeder einzelne Meßeinsatz z.B. durch Vergabe einer individuellen Identifikationsnummer vom Basisgerät erkenn bar. Weiterhin ist als Information das Herstellungsdatum des Meßeinsatzes gespeichert. Über derartige Informationen ist es möglich, daß das Basisgerät erkennt, ob ein Meßeinsatz, dessen Meßelektroden einem gewissen Alterungs- und Verschleißprozeß ausgesetzt sind, noch für eine einwandfreie Messung tauglich ist oder ob er ausgesondert werden sollte. Diese Kriterien sind vom Basisgerät über die Anzeige der jeweiligen Bedienungsperson mitteilbar.

Anspruch 1 lehrt auch eine Anordnung der Durchflußmeßzelle im Meßeinsatz, durch die deren Meßelektroden besonders sicher und geschützt gegen äußere Einflüsse, wie Stöße, Erschütterungen oder dergleichen gehalten sind. Damit ist die erfindungsgemäße Vorrichtung besonders geeignet für den rauhen Praxiseinsatz in Notarzt- oder Rettungswagen.

Durch die in Anspruch 2 angegebene Maßnahme wird das Einbringen der Probe, bei der es sich beispielsweise um mit einer Spritze oder kapillar entnommenes Vollblut, um Kapillarblut, Serum, Urin oder dergleichen handeln kann, in den Meßeinsatz erleichtert.

Der Anspruch 1 kennzeichnet auch Ausbildungen der erfindungsgemäßen Vorrichtung, die auf den Schutz von Bedienungspersonen der Vorrichtung vor Infektionen abzielt. Durch den in den Meßeinsatz integrierten, abgeschlossenen Proben-Sammelbehälter, der in Richtung des Durchflußkanals durch ein Rückschlagventil gegen Flüssigkeitsaustritt gesichert ist, werden die Probenflüssigkeiten in einem hermetisch abgeschlossenen Raum gesammelt und kommen nicht mehr in Kontakt mit der Außenwelt. Ist der Proben-Sammelbehälter im Meßeinsatz gefüllt, so muß letzterer lediglich in üblicher Weise entsorgt werden. Da der Meßeinsatz selbst mit keinen aktiven Bauelementen versehen ist, kann dies zu vertretbaren Kosten erfolgen.

Durch die in Anspruch 3 vorgesehene Tastatur am Basisgerät können meßrelevante Parameter oder patientenrelevante Informationen, wie beispielsweise Name, Adresse, äußere Umstände bei der Probenentnahme (Unfall, ect.) oder pathologische Befunde eingegeben werden, die zusammen mit den Analysewerten im Basisgerät gespeichert und/oder über die am Basisgerät vorgesehene Schnittstelle (Anspruch 4) zum Anschluß eines Datenverarbeitungsgerätes oder eines Datenverarbeitungs-Peripheriegerätes, wie beispielsweise eines Druckers zu Dokumentationszwecken einem rechnergestützten Patientendokumentationssystem in einer Klinik übergeben werden können.

Durch die im Anspruch 5 angegebene Tragbarkeit und den netzunabhängigen Betrieb der erfindungsgemäßen Vorrichtung ist diese besonders geeignet zum Einsatz in Notarzt- oder Rettungswagen. Natürlich kann das Gerät auch stationär und netzabhängig betrieben werden.

Weitere Merkmale, Einzelheiten und Vorteile der Erfindung sind der nachfolgenden Beschreibung entnehmbar, in der ein Ausführungsbeispiel anhand der beiliegenden Figuren näher erläutert wird.

Es zeigen:
- **Fig. 1**: eine perspektivische Darstellung der Vorrichtung mit aus dem Basisgerät herausgezogenen Meßeinsatz,
- **Fig. 2**: einen vertikalen Längsschnitt durch den Meßeinsatz gemäß Fig. 1 und
- **Fig. 3**: ein schematisches Blockdiagramm der geräteinternen Bauteile.

Eine erfindungsgemäße Vorrichtung zum Messen der lonenkonzentration in fließfähigen Medien insbesondere in Körperflüssigkeiten, wie Vollblut, Kapillarblut, Serum oder Urin besteht aus einem Basisgerät 1 und einem Meßeinsatz 2. Das Basisgerät 1 weist ein im wesentliches quaderförmiges Gehäuse 3 auf, dessen rückwärtiges Ende pultartig ausgebildet ist und dort im Bereich seiner geneigten Oberseite eine Flüssigkristall-Anzeigeeinheit 4 aufweist. Vor dem pultartigen Ende des Gehäuses 3 ist auf dessen Oberseite 5 eine Tastatur 6 zur Eingabe von meßrelevanten Parametern oder patientenrelevanten Informationen angeordnet.

In der Vorderseite 7 des Basisgerätes 1 ist seitlich ein Schacht 8 zum Einsetzen des Meßeinsatzes 2 vorgesehen.

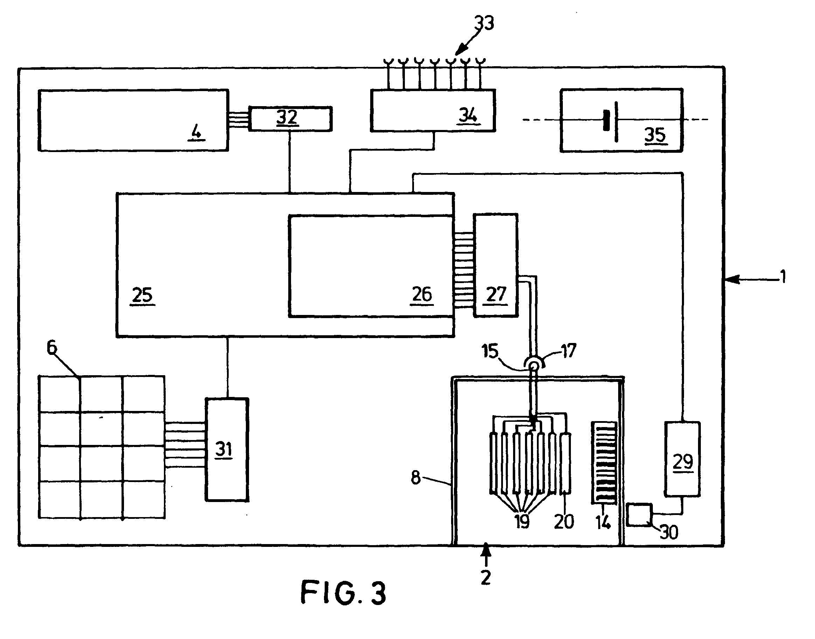

Der in seiner Außenform der Innenform des Schachtes 8 angepaßte Meßeinsatz 2 weist ein quaderförmiges Gehäuse 9 auf, in dessen Vorderseite 10 eine Griffmulde 11 eingeformt sowie oberhalb davon eine Einspritztülle 12 angeordnet ist. Auf seiner Oberseite 13 ist parallel zu der in Einschubrichtung E des Meßeinsatzes 2 in den Schacht 8 verlaufenden Seitenkante ein Strichcode-Aufkleber 14 aufgebracht. Im Bereich der oberen, in Einschubrichtung E weisenden Kante des Meßeinsatz-Gehäuses 9 steht von diesem eine Steckfahne 15 mit elektrischen Kontakten 16 ab, die beim Einsetzen des Meßeinsatzes 2 in das Basisgerät 1 mit Kontakten einer entsprechenden, in Fig. 3 schematisch dargestellten Steckbuchse 17 kontaktieren.

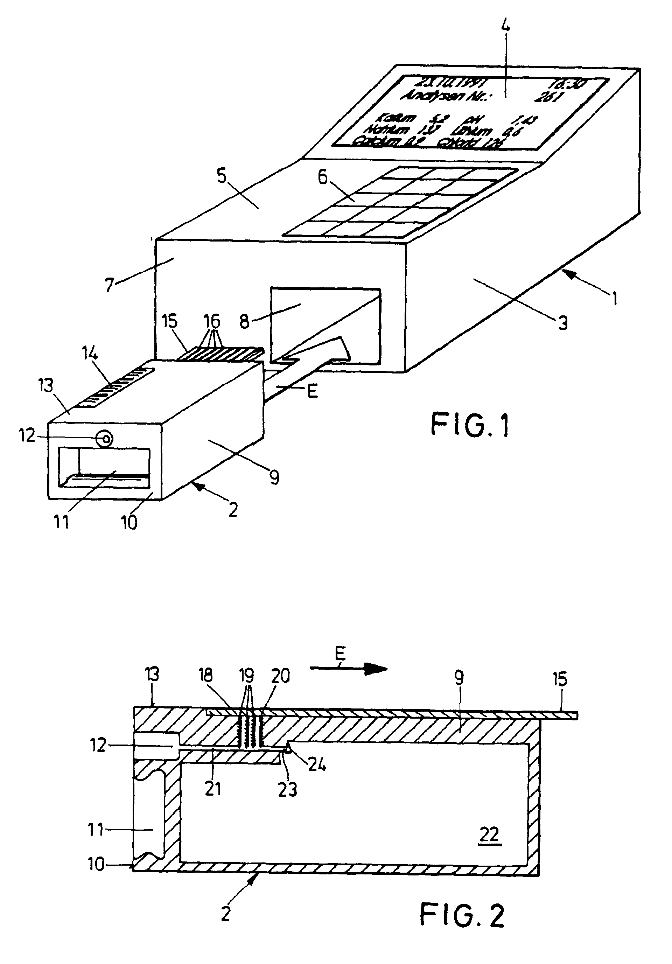

Der innere Aufbau des Meßeinsatzes 2 ist aus Fig. 2 erkennbar. Unterhalb seiner Oberseite 13 ist in seine Wandstruktur die Durchflußmeßzelle 18 eingelassen, die beispielsweise sechs Meßelektroden 19 und eine Bezugselektrode 20 aufweist. Jede Meßelektrode 19 ist auf jeweils eine lonenart abgestimmt. So können mit dem Meßeinsatz beispielsweise K⁺-, N⁺-, Ca⁺⁺-, Li⁺-, Cl⁻-Ionen und der pH-Wert gemessen werden. Jede Elektrode 19,20 steht über Leiterbahnen, die in Fig. 2 direkt unterhalb der Oberseite 13 des Meßeinsatz-Gehäuses 9 schematisch dargestellt sind, mit jeweils einem Kontakt 16 an der Steckfahne 15 in Verbindung.

Die Durchflußmeßzelle 18 steht in Fluidverbindung mit dem außenseitig in die Einspritztülle 12 des Meßeinsatzes 2 mündenden Durchflußkanal 21, der innenseitig in einen abgeschlossenen Proben-Sammelbehälter 22 mündet, der den Hauptteil des Gehäusevolumens des Meßeinsatzes 2 ausfüllt. An der innenseitigen Mündungsöffnung 23 des Durchflußkanals 21 ist ein Rückschlagventil 24 angeordnet, das sich in Richtung Proben-Sammelbehälter 22 öffnet, wenn eine Probe der zu messenden Flüssigkeit über die Einspritztülle 12 in den Durchflußkanal 21 eingespritzt wird. Nach dem Einbringen der Probe schließt das Rückschlagventil 24, damit kein Flüssigkeitsrücklauf aus dem Proben-Sammelbehälter 22 nach außen erfolgen kann.

In Fig. 3 ist schematisch der innere Aufbau des Basisgerätes 1 und des Meßeinsatzes 2 gezeigt.

Zur Steuerung der gesamten Vorrichtung dient eine zentrale Steuereinheit 25 in Form eines Mikroprozessors mit entsprechenden Arbeits-, Daten- und Programmspeichern sowie einer zentralen Prozeßrecheneinheit (CPU). Hard- und/oder softwaremäßig kann in die zentrale Steuereinheit 25 die Meßwerterfassungs- und Auswerteeinrichtung 26 integriert sein, mit der die Meßelektroden 19 und die Bezugselektrode 20 jeweils über als Block in Fig. 3 dargestellte A/D-Wandler 27 elektrisch verbunden sind. Weiterhin ist die zentrale Steuereinheit 25 mit dem Treiber 29 der Strichcode-Leseeinrichtung 30 verbunden, die beim Einschieben des Meßeinsatzes 2 in den Schacht 8 des Basisgerätes 1 den Strichcode auf dem Strichcode-Aufkleber 14 abtastet und die entsprechend codierten Informationen über den Treiber 29 der Steuereinheit 25 zuführt. Diese Informationen, betreffend beispielsweise die von den Meßelektroden 19 faßbaren lonenarten, werden der Meßwerterfassungs- und Auswerteeinheit 26 zugeführt, damit letztere die entsprechenden Parameter zur Auswertung der von den Meßelektroden 19 herangeführten Potentialwerte zur Verfügung hat.

Weiterhin ist mit der zentralen Steuerheit 25 die Tastatur 6 über den Tastaturtreiber 31 sowie die Flüssigkristall-Anzeigeeinheit 4 über den LCD-Treiber 32 verbunden.

Das Basisgerät 1 ist darüberhinaus mit einer Schnittstelle 33 zum Anschluß eines Personalcomputers oder eines Druckers verbunden Die Schnittstelle 33 ist über den Schnittstellentreiber 34 ebenfalls mit der Steuereinheit 25 verbunden.

Zur netzunabhängigen Stromversorgung des Basisgerätes 1 dient die Batterie 35.

Zusammenfassend können zum Basisgerät 1 verschiedenste Meßeinsätze 2 mit an unterschiedlichste Aufgaben angepaßten Elektroden 19,20 bereitgestellt werden, die von der Bedienungsperson entsprechend dem Anwendungszweck ausgesucht und lediglich in das Basisgerät 1 eingeschoben werden müssen. Als Anwendungsbereiche sind die Notfallmedizin - z.B. in einem Notarztwagen -, die Dialyse, Anästhesie, Intensivmedizin auf der Intensivstation und dergleichen zu nennen, wobei zwischen Meßeinsätzen 2 oder -kartuschen zur Bestimmung von Elektrolyten und/oder Enzymen und/ oder Blutgasen usw frei gewählt werden kann. Die Meßeinsätze tragen dann in jeweils abgestimmter Weise Elektroden 19,20 für potentiometrische und/oder ampereometrische Messungen und/oder Elektroden zu Bikarbonat -, d.h. pCO₂ -, Glukose - o.ä. Bestimmungen.

## Patentansprüche

1. Vorrichtung zum Messen der Ionenkonzentration in fließfähigen Medien, insbesondere in Körperflüssigkeiten, mit einer Durchflußmeßzelle (18) umfassend mehrere, auf die jeweils zu messende Ionenart abgestimmte Meßelektroden (19), mit einer Meßwerterfassungs- und Auswerteeinrichtung (26), die mit den Meßelektroden (19) elektrisch verbunden ist, mit einer Anzeigeeinheit (4) zur Anzeige der von der Meßwerterfassungs- und Auswerteeinrichtung (26) ermittelten Ionenkonzentration und mit einer Steuereinheit (25) zur Steuerung der vorrichtungsinternen Betriebsabläufe,
bei der die Durchflußmeßzelle (18) in einem für mehrere Proben-flüssigkeiten verwendbaren Meßeinsatz (2) angeordnet ist, der in ein die Meßwerterfassungs- und Auswerteeinrichtung (26), die Anzeigeeinheit (4) sowie die Steuereinheit (25) umfassendes Basisgerät (1) unter Herstellen einer elektrischen Verbindung zwischen den Elektroden (19) im Meßeinsatz (2) und der Meßwerterfassungs- und Auswerteeinrichtung (26) auswechselbar einsetzbar ist,
bei der der Meßeinsatz (2) eine Kodierung (14) aufweist, die von einer Code-Leseeinrichtung (30) im Basisgerät (1) lesbar ist,
bei der die Durchflußmeßzelle (18) in die Wandstruktur des Meßeinsatzes (2) eingelassen ist und in Fluidverbindung mit einem außenseitig offenen Durchflußkanal (21) in dieser Wandstruktur für eine Probe des zu messenden Mediums steht, und
bei der der Durchflußkanal (21) innenseitig in einen Behälter (22) mündet,
**dadurch gekennzeichnet,**
**daß** die Durchflußmeßzelle (18) eine Bezugselektrode (20) und die Meßelektroden (19) umfaßt, mit denen die Meßwerterfassungs- und Auswerteeinrichtung (26) elektrisch verbunden sind,
**daß** der Behälter ein abgeschlossener Proben-Sammelbehälter (22) ist und an der innenseitigen Mündungsöffnung (23) des Durchflußkanals (21) ein Rückschlagventil (24) angeordnet ist,
**daß** zur Kodierung ein in den Meßeinsatz (2) integrierter PROM-Speicher verwendet ist.

2. Vorrichtung nach Anspruch 1, **dadurch gekennzeichnet, daß** die außenseitige Mündung des Durchflußkanals (21) als Einspritz-Tülle (12) ausgebildet ist.

3. Vorrichtung nach einem der Ansprüche 1 und 2, **dadurch gekennzeichnet, daß** das Basisgerät (1) mit einer Tastatur (6) zur Eingabe von Parametern und Informationen versehen ist.

4. Vorrichtung nach einem der Ansprüche 1 bis 3, **dadurch gekennzeichnet, daß** das Basisgerät (1) eine Schnittstelle (33) zum Anschluß eines Dateriverarbeitungsgerätes oder Datenverarbeitungs-Peripheriegerätes aufweist.

5. Vorrichtung nach einem der Ansprüche 1 bis 4, **dadurch gekennzeichnet, daß** die Vorrichtung tragbar und netzunabhängig betreibbar ist.

## Claims

1. Apparatus for measuring the ion concentration in flowable media, in particular in body fluids, with a flow cell (18) comprising a plurality of measuring electrodes (19) adapted to the type of ion to be measured respectively, with a data detection and evaluation unit (26), which is electrically connected to the measuring electrodes (19), with a display (4) to display the ion concentrations detected by the data detection and evaluation unit (26) and with a control unit (25) to control the operational processes within the apparatus, in which the flow cell (18) is arranged in a measuring insert (2) which can be used for a plurality of samples of fluids and can be replaceably inserted into a base instrument (1) comprising the data detection and evaluation unit (26), the display (4) as well as the control unit (25), whereby an electric connection is produced between the electrodes (19) in the measuring insert (2) and the data detection and evaluation unit (26), in which the measuring insert (2) has an encoding (14), which is readable by a code reader (30) in the base instrument (1), in which the flow cell (18) is provided in the wall structure of the measuring insert (2) and is in fluid connection with a flow channel (21) open to the outside in this wall structure for a sample of the medium to be measured, and in which the flow channel (21) opens to the inside into a container (22), **characterised in that** the flow cell (18) comprises a reference electrode (20) and the measuring electrodes (19) to which the data detection and evaluation unit (26) are electrically connected, **in that** the container is a closed sample container (22) and a check valve is arranged at the inside discharge opening (23) of the flow channel (21), **in that** a PROM memory integrated in the measuring insert (2) is used for coding.

2. Apparatus according to claim 1, **characterised in that** the outside opening of the flow channel (21) is in the form of an injection nozzle (12).

3. Apparatus according to either of claims 1 or 2, **characterised in that** the base instrument (1) is provided with a keyboard (6) for parameters and data to be entered.

4. Apparatus according to any one of the claims 1 to 3 **characterised in that** the base instrument (1) has an interface (33) for the connection of a data processing device or a data processing peripheral device.

5. Apparatus according to any one of the claims 1 to 4, **characterised in that** the apparatus is portable and can be operated off the line.

## Revendications

1. Appareil pour mesurer la concentration des ions dans des milieux capables de s'écouler, en particulier dans des liquides corporels, appareil qui comprend une cellule de mesure de débit (18) comportant plusieurs électrodes de mesure (19) accordées respectivement aux types d'ions à mesurer, comprenant un dispositif de détection et d'évaluation des valeurs de masure (26), lequel est relié électriquement aux électrodes de mesure (19), comprenant une unité d'affichage (4) pour afficher la concentration des ions déterminée par le dispositif de détection et d'évaluation de valeurs de mesure (26), et comprenant une unité de commande (25) pour commander le déroulement des opérations internes de l'appareil, la cellule de mesure de débit (18) étant agencée dans un insert de mesure (2) utilisable pour plusieurs liquides à tester, ledit insert pouvant être mis en place de façon interchangeable dans un appareil de base (1) qui comprend le dispositif de détection et d'évaluation de valeurs de mesure (26), l'unité d'affichage (4), ainsi que l'unité de commande (25), en établissent une liaison électrique entre les électrodes (19) dans l'insert de mesure (2) et le dispositif de détection et d'évaluation de valeurs de mesure (26),
l'insert de mesure (2) présentant un codage (14), lequel peut être lu par un dispositif de lecture de code (30) dans l'appareil de base (1),
la cellule de mesure de débit (18) étant ménagée dans la structure de paroi de l'insert de mesure (2), et étant en liaison de fluide avec un canal de traversée (21), ouvert vers l'extérieur, dans cette structure de paroi, pour un échantillon du milieu à mesurer,
le canal de traversée (21) débouchant à l'intérieur dans un réservoir (22),
**caractérisé en ce que**,
la cellule de mesure de débit (18) comprend une électrode de référence (20) et les électrodes de mesure (19) auxquelles est relié électriquement le dispositif de défection et d'évaluation des valeurs de mesure,
**en ce que** le réservoir est un réservoir de collecte d'échenillions fermé (22) et une soupape anti-retour (24) est agencée sur l'ouverture d'embouchure intérieure (23) du canal de traversée (21),
**en ce que** pour le codage, on utilise une mémoire PROM intégrée dans l'insert de mesure (2).

2. Appareil selon la revendication 1, **caractérisé en ce que** l'embouchure extérieurs du canal de traversée (21) est réalisée sous forme d'une douille d'injection (12).

3. Appareil selon l'une des revendications 1 à 2, **caractérisé en ce que** l'appareil de base (1) est pourvu d'un clavier (6) pour la saisie de paramètres et d'informations.

4. Appareil selon l'une des revendications 1 à 3, **caractérisé en ce que** l'appareil de base (1) comporte une interface (33) pour le raccordement d'un appareil de traitement des données ou d'un appareil périphérique de traitement des données.

5. Appareil selon l'une des revendications 1 à 4, **caractérisé en ce que** l'appareil est portable et peut être utilisé indépendamment du réseau électrique.
